# EUROPÄISCHE PATENTANMELDUNG

(11) **EP 1 982 602 A1**
(43) Veröffentlichungstag der Anmeldung: **22.10.2008**
(21) Anmeldenummer: 08007475.0
(22) Anmeldetag: 17.04.2008
(51) Int. Cl.: A23K 1/18, A23K 1/16, A23K 1/175, A61P 43/00

(54) **Tierfutterzusatz zur Behandlung von Tierzähnen**

(30) Priorität: 19.04.2007 DE 102007018488; 09.05.2007 DE 102007021672
(71) Anmelder: Fuss, Jochen, 45472 Mühlheim an der Ruhr (DE)
(72) Erfinder: Fuss, Jochen, 45472 Mühlheim an der Ruhr (DE)
(74) Vertreter: Schulte, Jens Michael

(57) **Zusammenfassung**

Einem Futtermittel für Tiere mit Zusätzen wie Stroh, Heu, Hafer, Gerste, Weizenkleie, Mehl, Mais, Gemüse oder Obst wird ein Zusatz aus abrasiven Material, zweckmäßigerweise aus Mineralien beigefügt. Dies führt zu einer längeren Lebensdauer, einer besseren Futterverwertung und erheblich seltener auftretenden Problemen des gesamten Kauapparates.

## Beschreibung

Die Erfindung betrifft ein Futtermittel für Tiere mit Zusätzen wie Stroh, Heu, Hafer, Gerste, Weizenkleie, Mehl, Mais, Gemüse und/oder Obst.

In erster Linie ist dabei an Futtermittel für Tiere gedacht, deren Zähne sich durch ein ausgeprägtes Wachstum auszeichnen. Es geht also vor allem um Tiere, bei denen, bezogen auf die Lebensdauer, das Wachstum der Zähne sehr lange anhält oder sogar das ganze Leben fortdauert. Insbesondere ist dies für Pferde und Esel bzw. deren Mischformen, sowie Rinder, speziell Milchvieh der Fall. Heutzutage werden für diese Tiere die Futtermittel zumeist industriell hergestellt, wobei natürlich auf eine ausgewogene, nährstoffreiche und gesunde Ernährung dieser Tiere Wert gelegt wird. Von den Umständen und Zusammensetzungen der natürlichen sind diese industriellen Futter aber einigermaßen weit entfernt. Dies bezieht sich speziell auf die Wirkung eines Futters auf die Zähne des Tiers, welche, wie so häufig, unter natürlichen Umständen besonders vorteilhaft ist, was bisher bekannte Futter aber nicht zu imitieren oder leisten fähig sind. Insbesondere gibt es kein Futtermittel oder keine Zusammensetzung hierfür, die dafür geeignet wäre, dahingehend positiven Einfluss auf das Wachstum der Zähne der Tiere zu nehmen, dass dieses den theoretischen Bedingungen in freier Wildbahn nachempfunden wäre, gerade was auch das lang andauernde bzw. lebenslange Wachstum der Zähne bei manchen Tieren betrifft.

Hiervon ausgehend stellt sich der vorliegenden Erfindung die Aufgabe, ein Futtermittel bzw. einen Zusatz für ein solches zu schaffen, das sich positiv auf die Ausbildung und das Wachstum der Zähne der Tiere auswirkt.

Diese Aufgabe wird dadurch gelöst, dass das Futtermittel einen Zusatz aus einem abrasiven Material enthält.

Es handelt sich dabei um einen Futtermittelzusatz bzw. ggf. auch ein eigenständiges Futterformat, insbesondere für das Futter von Pferden und Eseln, bzw. deren Mischformen sowie Rinder, bei dem durch gezielte Zugabe eines abrasiven Materials nunmehr eine regulierende Wirkung auf die Zähne der Tiere bzw. deren Wachstum ausgeübt werden kann. Unter gezielter Zugabe dieses abrasiven Materials wird dabei in gewissen Umfang das unter natürlichen Bedingungen zu findende Futter für diese Tiere nachempfunden. Die Abnutzung, Abschabung bzw. Abtragung und die damit einher gehende Regulierung bezüglich des Nachwachsens und Nachschiebens der Zähne in den Gebissen dieser Tiere wirken sich dabei vorteilhaft auf die Gesundheit der Tiere aus. Gebissabnormalitäten, deren Auftreten die Tiere einschränkt und behindert und im Übrigen die Aufnahme und Verwertung des Futters beeinträchtigen, werden damit erfolgreich vermieden.

Die Bedürfnisse eines Tiers bezüglich Alter, Gesundheitszustand oder anderer Umstände berücksichtigend, wird vorgeschlagen, dass der abrasive Zusatz eine Mohs-Härte von ca. 5-9 aufweist. Damit wird das Tier quasi zu schleifenden oder schmirgelnden Bewegungen seines Gebisses gezwungen, was sich wiederum vorteilhaft auf das Wachstum und die Entwicklung der Zähne erweist.

Eine besonders geeignete Ausführung eines Futtermittels sieht dabei vor, dass der abrasive Zusatz eine Mohs-Härte von ca. 6-7 aufweist, um die angesprochenen Wirkungen bezüglich des positiven Einflusses auf die Zähne der Tiere zu erreichen.

Besonders gute Effekte können erreicht werden, wenn es sich bei dem Zusatz um ein Mineral handelt. Dies gilt einerseits in Hinblick auf eine möglichst optimale Nachbildung der natürlichen Umwelt für Tiere wie Pferde, Esel oder Rinder, mit denen eine Anlehnung an natürliche Umstände erreicht wird. Andererseits sind solche Materialien für die Tiere gut verträglich und lassen sich im Übrigen auch herkömmlichem Tierfutter auf besonders geeignete und leichte Weise beifügen. Dies kann industriell geschehen, wobei auch die Art, Zusammensetzung und Dosierung individuell für Tierarten, -rassen oder bestimmte Altersgruppen etwa variiert werden kann.

Als besonders geeignetes und auch kostengünstiges Material hat es sich erwiesen, wenn es sich bei dem Zusatz um einen Sand, vorzugsweise einen Quarzsand handelt. Alternativ dazu ist an einen Zusatz aus bzw. mit Korund, Bimsstein und/oder Feldstein gedacht.

Als gleich in mehrerlei Hinsicht vorteilhaft anzusehen ist es, wenn es sich bei dem Zusatz um ein pflanzliches Material handelt. Diese Variante der Erfindung bringt nicht nur Kostenvorteile mit sich, sondern ist aufgrund des Einsatzes eines nachwachsenden natürlichen Materials auch in ökologischer Hinsicht optimal. Nicht zuletzt sind diese natürlichen abrasiven Stoffe als besonders verträglich anzusehen. Gedacht ist an Pflanzen diverser Art, Hölzer oder auch Gemüse wie etwa Mais.

Weiterhin ist vorgesehen, dass es sich bei dem Zusatz um ein Mehl oder einen Schlamm, also um weitgehend natürliche Zusätze handeln kann. Diese können in gerundeter oder auch gebrochener Form dem Futter zugefügt werden, in den unterschiedlichsten Mahlungen, Härten und Körnungen.

Ein weiterer Vorschlag sieht vor, dass es sich bei dem Zusatz um Hölzer handelt. Diese können in der Form von Rinden, Wurzeln, Spänen oder in anderem Zustand dem Futter beigemengt werden. Selbstverständlich ist es in diesem Zusammenhang auch denkbar, die zuvor angesprochenen Ausführungsformen der Erfindung beliebig miteinander zu kombinieren.

Zielgerichtet kann dies erfolgen, wenn es sich bei dem Zusatz im Holzfasern und/oder Pflanzenfasern handelt. Eine hohe abrasive Wirkung entfalten dabei vor allem harte Pflanzenstengel, wie z.B. die von Luzemen.

Eine Art von besonders kompaktem Zusatz für ein Futtermittel zeichnet sich dadurch aus, dass die Holzfasern und/oder Pflanzenfasern gefaltet sind. Eine bis zu achtmal ausgeführte Faltung ist als besonders vorteilhaft anzusehen.

Eine weitere vorteilhafte Ausführungsform der Erfindung ist verwirklicht, wenn es sich bei dem abrasiven Zusatz um Zähne handelt. Diese stammen vorzugsweise von verstorbenen Tieren und bringen daher die hervorragende Eigenschaft mit sich, von ihrer Zusammensetzung und von ihrem Härtegrad den Zähnen der zu fütternden Tiere zu entsprechen. Dies bewirkt eine besonders effektive und gleichmäßige Beanspruchung des Gebisses beim Kauen.

Der abrasive Zusatz kann dem übrigen Futtermittel auf unterschiedlichste Art beigefügt werden. Dies ist z. B. der Fall, wenn der abrasive Zusatz dem Futtermittel beigemengt ist, wenn es sich also um einen Zusatz in fester Form handelt, der dem Futter bei- oder nach der Herstellung beigemengt wird.

Eine Variante hierzu sieht vor, dass der abrasive Zusatz dem Futtermittel in der Form von Pellets zugefügt ist. Bei dieser Variante der Erfindung wird die Tiernahrung bzw. deren Ergänzung zu Pellets gepresst, die in Pelletieranlagen mit großem Druck hergestellt werden.

Eine weitere Ausführungsform der Erfindung sieht vor, dass der abrasive Zusatz dem Futtermittel beigemischt ist, also in flüssiger bzw. zähflüssiger Form dem übrigen Futtermittel zugefügt wird.

Die Erfindung zeichnet sich insbesondere dadurch aus, dass ein Futtermittel für Tiere, insbesondere für Pferde, Esel oder Rinder geschaffen ist, welches einen Zusatz oder mehrere verschiedene Zusätze aus einem abrasiven und damit das Gebiss des Tieres positiv beeinflussenden Material enthält. Damit wird ein bisher bestehender Nachteil industriell hergestellter Futtermittel ausgeglichen, die sich vor allem auf das Wachstum der Zähne dieser Tiere in keiner Weise regulierend und damit negativ ausgewirkt haben. Erfindungsgemäß besteht das Futter aus Hartstoffkömern bzw. wird es anderen Futtermittelkomponenten wie Heu, Hafer, Stroh, Gerste, Gemüse oder Obst auf geeignete Weise beigefügt. Durch das Ausüben einer entsprechenden Schleif- oder Schmirgelbewegung auf die Zähne, bewirkt durch den abrasiven Zusatz, der das Tier zu den entsprechenden Bewegungen verleitet bzw. sogar zwingt, wird die eigentlich von der Natur vorgesehene Abnutzung, Abschabung oder Abtragung der auch nachwachsenden oder sich nachschiebenden Zähne unterstützt. Eine damit einhergehende längere Lebensdauer und eine bessere Futterverwertung führen zu erheblich seltener auftretenden Problemen des kompletten Kauapparates. Der Futtermittelindustrie und der milchproduzierenden Wirtschaft hilft der erfindungsgemäße Zusatz für ein Futtermittel durch Senkung der Produktionskosten. Sport-, Freizeit- und Berufsreiter erfreuen sich einer längeren Vitalität und Lebensdauer und längere sportliche, freizeitliche oder wirtschaftliche Nutzung der Tiere. Der Zusatz kann dem übrigen Futtermittel in unterschiedlichster Form, vorzugsweise in Form von Mineralien, zugeführt werden und zwar durch Beimengung, Vermischung, Anhaftung, Mitverpressung oderverbackung, sodass in jedem Fall auch gewährleistet ist, dass die Tiere den abrasiven Zusatz mit der Nahrung in geeigneter Weise aufnehmen können. Zusätze auf pflanzlichen und/oder mineralischen Bestandteilen werden als besonders geeignet angesehen. Vorteilhaft ist eine gewisse Härte dieser Zusätze, wie sie z.B. Sande, Pflanzenstengel oder Fasern von Pflanzen und Hölzern mit sich bringen.

## Patentansprüche

1. Futtermittel für Tiere mit Zusätzen wie Stroh, Heu, Hafer, Gerste, Weizenkleie, Mehl, Mais, Gemüse und/oder Obst,
**dadurch gekennzeichnet,**
**dass** das Futtermittel einen Zusatz aus einem abrasiven Material enthält.

2. Futtermittel nach Anspruch 1,
**dadurch gekennzeichnet,**
**dass** der abrasive Zusatz eine Mohs-Härte von ca. 5-9, vorzugsweise von ca. 6-7 aufweist.

3. Futtermittel nach Anspruch 1,
**dadurch gekennzeichnet,**
**dass** es sich bei dem Zusatz um ein Mineral handelt.

4. Futtermittel nach Anspruch 3,
**dadurch gekennzeichnet,**
**dass** es sich bei dem Zusatz um einen Sand, vorzugsweise einen Quarzsand handelt.

5. Futtermittel nach Anspruch 3,
**dadurch gekennzeichnet,**
**dass** es sich bei dem Zusatz um ein Korund, Bimsstein und/oder Feldstein handelt.

6. Futtermittel nach Anspruch 1,
**dadurch gekennzeichnet,**
**dass** es sich bei dem Zusatz um ein pflanzliches Material handelt.

7. Futtermittel nach Anspruch 6,
**dadurch gekennzeichnet,**
**dass** es sich bei dem Zusatz um ein Mehl handelt.

8. Futtermittel nach Anspruch 1,
**dadurch gekennzeichnet,**
**dass** es sich bei dem Zusatz um einen Schlamm handelt.

9. Futtermittel nach Anspruch 6,
**dadurch gekennzeichnet,**
**dass** es sich bei dem Zusatz um Hölzer handelt.

10. Futtermittel nach Anspruch 9,
**dadurch gekennzeichnet,**
**dass** es sich bei dem Zusatz um Holzfasern und/oder Pflanzenfasern handelt.

11. Futtermittel nach Anspruch 10,
**dadurch gekennzeichnet,**
**dass** die Holzfasern und/oder Pflanzenfasern gefaltet sind.

12. Futtermittel nach Anspruch 1,
**dadurch gekennzeichnet,**
**dass** es sich bei dem abrasiven Zusatz um Zähne in zerkleinerter Form handelt.

13. Futtermittel nach Anspruch 1,
**dadurch gekennzeichnet,**
**dass** der abrasive Zusatz dem Futtermittel beigemengt ist.

14. Futtermittel nach Anspruch 1,
**dadurch gekennzeichnet,**
**dass** der abrasive Zusatz dem Futtermittel in der Form von Pellets zugefügt ist.

15. Futtermittel nach Anspruch 1,
**dadurch gekennzeichnet,**
**dass** der abrasive Zusatz dem Futtermittel beigemischt ist.
